# EUROPÄISCHE PATENTSCHRIFT

(11) **EP 0 433 615 B1**
(45) Veröffentlichungstag und Bekanntmachung des Hinweises auf die Patenterteilung: **08.12.1993**
(21) Anmeldenummer: 90120672.2
(22) Anmeldetag: 29.10.1990
(51) Int. Cl.: C07C 255/46, C07C 253/10

(54) **Verfahren zur Herstellung von 1,3,3-Trimethyl-5-oxo-cyclohexan-carbonitril**
Process for the preparation of 1,3,3-trimethyl-5-oxo-cyclohexanecarbonitrile
Procédé pour la préparation de triméthyl-1,3,3, oxo-5 cyclohexane carbonitrile

(30) Priorität: 21.12.1989 DE 3942371
(43) Veröffentlichungstag der Anmeldung: 26.06.1991
(73) Patentinhaber: Degussa Aktiengesellschaft, 60311 Frankfurt (DE)
(72) Erfinder: Huthmacher, Klaus, Dr., W-6460 Gelnhausen (DE); Schmitt, Hermann, W-6451 Rodenbach (DE)

(56) Entgegenhaltungen:
- DE-B- 1 240 854
- DE-C- 1 085 871
- PATENT ABSTRACTS OF JAPAN, unexamined applications, C Sektion, Band 6, Nr. 208, 20. Oktober 1982 THE PATENT OFFICE JAPANESE GOVERNMENT Seite 121 C 130

## Beschreibung

Die Erfindung richtet sich auf ein Verfahren zur Herstellung von 1,3,3-Trimethyl-5-oxo-cyclohexan-carbonitril durch Anlagerung von Cyanwasserstoff an Isophoron in Gegenwart einer alkalisch wirkenden Alkaliverbindung als Katalysator bei 100 bis 160 °C. Das erfindungsgemäße Verfahren zur Herstellung von 1,3,3-Trimethyl-5-oxo-cyclohexan-carbonitril, das auch als 3-Cyano-3,5,5-trimethyl-cyclohexanon oder kurz Isophoronnitril bezeichnet wird, zeichnet sich durch die hohe Wirksamkeit des verwendeten Katalysators aus.

Die Addition von Cyanwasserstoff an Isophoron ist an sich lange bekannt. Die Umsetzung erfolgt basekatalysiert bei erhöhter Temperatur.

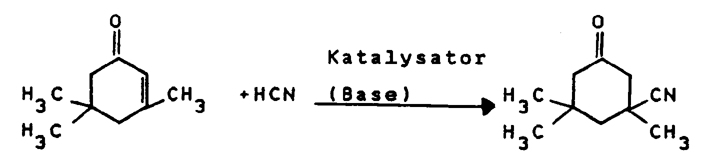

Gemäß dem Verfahren der DE-AS 10 85 871 werden Isophoron und Cyanwasserstoff bei 125 bis 275 °C in Gegenwart eines stark alkalischen Cyanidionen bildenden Katalysators und vorzugsweise eines stark polaren Lösungsmittels, wie Dimethylformamid oder Dimethylacetamid, umgesetzt. Die Cyanwasserstoffsaure wird im wesentlichen mit der Geschwindigkeit zugegeben, mit der sie sich umsetzt. Als Katalysator werden u. a. Alkalimetalle und ihre Carbonate, Alkali- und Erdalkalialkoholate, -oxide, -hydroxide und -cyanide, ferner Amine und quaternäre Ammoniumbasen genannt. Die Katalysatorkonzentration wird mit 0,1 bis 20 Gew.-%, bezogen auf das Reaktionsgemisch, angegeben. Das Reaktionsgemisch wird nach der Umsetzung mit Phosphorsäure versetzt, um den Katalysator zu neutralisieren, und anschließend destillativ aufgearbeitet. Wie aus dem relevanten Beispiel dieses Dokuments hervorgeht, mußte der Cyanwasserstoff sehr langsam zudosiert werden, was zu einer unbefriedigenden Raum-Zeit-Ausbeute führte. Nachteilig ist ferner, daß hier ein artfremdes Lösungsmittel mitverwendet werden mußte, wodurch der Aufwand für die Aufarbeitung des Reaktionsgemisches erhöht und Isophoronnitril nur in mittlerer Ausbeute und mäßiger Reinheit erhalten wurde.

Die DE-PS 12 40 854 richtet sich auf eine Verbesserung des vorgenannten Verfahrens, indem in Abwesenheit eines Lösungsmittels mit geringeren Katalysatormengen, nämlich 10⁻¹ bis 10⁻³ Gew.-%, bezogen auf das Reaktionsgemisch, eine höhere Ausbeute erreicht werden sollte. Die Reaktionszeit - 4 Stunden Verweilzeit im Hauptreaktor und je 1 Stunde in zwei Nachreaktoren gemäß Beispiel 2 der DE-PS 12 40 854 - konnte hierdurch aber nicht verkürzt werden.

Unter Bezugnahme auf die zuvor gewürdigte DE-AS 10 85 871 und DE-PS 12 40 854 versuchte die Anmelderin der JP-A 57-116038 die genannten Verfahren zu verbessern. Nach den in dem japanischen Dokument offenbarten Vergleichsversuchen, wurde unter Verwendung einer methanolischen NaOH-Lösung als Katalysator (0,9 ml 15 %ig pro 204 g Isophoron) nach einer gesamten Reaktionszeit von 4 ,5 Stunden eine Ausbeute von 53,7 % und unter Verwendung von K₂CO₃ als Katalysator (4,9 g pro 192,2 g Isophoron) und Mitverwendung von Dimethylformamid eine Ausbeute von 71,1 % erhalten. Die JP-A 57-116038 lehrt demgegenüber, Cyanwasserstoff in Gegenwart von basischen Katalysatoren, wie zum Beispiel Alkalimetallcyanide, -carbonate, -oxide, -hydroxide und -alkoholate, und Glykolen mit Isophoron zur Reaktion zu bringen. Die Glykole werden in der 1- bis 50-fachen Menge des Katalysators eingesetzt. Zwar werden in den Beispielen hohe Ausbeuten an Isophoronnitril angegeben, aber die Reaktionszeiten unter Verwendung von Natrium- bzw. Kaliumcarbonat oder Natriumcyanid liegen im Bereich der seinerzeit bereits vorbekannten Verfahren. Die Raum-Zeit-Ausbeute blieb also weiterhin unbefriedigend. Durch die Verwendung der Glykole gestaltet sich die Aufarbeitung aufwendiger und die Wirtschaftlichkeit wird gemindert.

Weitere Verfahren betreffen die Verwendung von quaternären Ammonium- oder Phosphoniumhydroxiden - vgl. JP-A 61-33157 - oder Diazabicycloalkenen - vgl. JP-A 61-33158 - als Katalysator. Diese Katalysatoren sind aber recht teuer.

In der DE-PS 12 40 521 wird ein Verfahren beschrieben, bei welchem die Umsetzung zwischen Isophoron und Cyanwasserstoff in Gegenwart von auf festen Trägern aufgebrachten alkalischen Katalysatoren bei 50 bis 350 °C erfolgt. Als Katalysatoren werden vorzugsweise Alkalihydroxide oder Alkalicyanide auf die Träger aufgebracht. Die Herstellung der Trägerkatalysatoren ist recht umständlich, die Durchführung des Additionsverfahrens aufwendig, da die HCN-Konzentration im Reaktionsgemisch niedrig gehalten werden muß, wozu die Blausäure in der Gasphase mit Stickstoff verdünnt wird. Das Verfahren erscheint somit trotz der angegeben hohen Ausbeuten für die Technik nicht wirtschaftlich.

Schließlich kann 1,3,3-Trimethyl-5-oxo-cyclohexan-carbonitril auch aus Isophoron und Alkalicyaniden in einem wäßrig-organischen Zweiphasensystem in Gegenwart eines Phasentransferkatalysators gewonnen werden - EP-B 028 179. Dieses Verfahren erfordert aber den Einsatz eines gegenüber Cyanwasserstoff teureren Alkalicyanids und einer nicht unbeträchtlichen Menge eines teuren Transferkatalysators; die Aufarbeitung der wäßrigen Phase führt zu einen erheblichen Salzanfall und damit Entsorgungsproblemen.

Die Aufgabe der vorliegenden Erfindung besteht nun darin, das gattungsgemäße Verfahren zur Herstellung von 1,3,3-Trimethyl-5-oxo-cyclohexan-carbonitril durch Anlagerung von Cyanwasserstoff an Isophoron in Gegenwart einer alkalisch wirkenden Alkaliverbindung als Katalysator bei 100 bis 160 °C so zu verbessern, daß bei einer Ausbeute von etwa 90 % und mehr eine höhere Raum-Zeit-Ausbeute resultiert. Das Verfahren sollte ferner einfach durchführbar und nicht auf die Verwendung teurer Katalysatoren und/oder Hilfsstoffe und/oder artfremder Lösungsmittel angewiesen sein.

Die Aufgabe wird dadurch gelöst, daß man Lithiumhydroxid als Katalysator verwendet und die Umsetzung in Gegenwart von 0,005 bis 5 Mol-%, bezogen auf Isophoron, durchführt.

Es wurde festgestellt, daß die katalytische Wirksamkeit von Lithiumhydroxid überraschenderweise jene der anderen Alkalihydroxide bei weitem übersteigt. Obgleich in den Dokumenten zum Stand der Technik alkalisch wirkende Alkaliverbindungen allgemein genannt wurden und in den dort ausgeführten Beispielen Hydroxide, Cyanide und Carbonate von Natrium und Kalium als Katalysatoren eingesetzt wurden, konnte hieraus nicht geschlossen werden, daß Lithiumhydroxid eine außergewöhnlich hohe katalytische Aktivität entfalten könnte.

Ganz offensichtlich war der überraschende Effekt von Lithiumhydroxid zuvor überhaupt nicht erkannt worden. Dieser Effekt folgt aus dem Vergleich der erfindungsgemaßen Beispiele mit den Vergleichsbeispielen 1 und 2 unter Verwendung von Natrium- bzw. Kaliumhydroxid als Katalysator; NaOH und KOH zeigen, wenn sie ohne zusätzliche Hilfsstoffe zum Einsatz kommen, nur eine mäßige Aktivität, und wirtschafliche Produktausbeuten sind hiermit nicht zu erzielen. Mit Lithiumhydroxid können demgegenüber Ausbeuten um und meist über 90 % erreicht werden - d. h., die als Nebenreaktion bekannte Polymerisation von Blausäure und Oligomerisierung von Isophoron werden praktisch vollständig vermieden.

Herausragend sind die mit LiOH erzielbaren außerordentlich kurzen Reaktionszeiten - unter vergleichbaren Bedingungen erfindungsgemäß 5 bis 15 Minuten, nach dem Stand der Technik 3 bis 4 Stunden und länger. Insgesamt läßt sich damit das erfindungsgemäße Verfahren mit hohen Raum-Zeit-Ausbeuten durchführen.

Lösungsmittel oder andere Hilfsstoffe sind im erfindungsgemäßen Verfahren im allgemeinen nicht erforderlich, jedoch wird im allgemeinen ein Überschuß von Isophoron gegenüber Cyanwasserstoff eingesetzt; ein Molverhältnis von Isophoron zu Cyanwasserstoff im Bereich von 1 ,1 zu 1 bis 5 zu 1, insbesondere 1,5 zu 1 bis 2,5 zu 1, wird bevorzugt.

Nicht vorsehbar war, daß die katalytische Aktivität von Lithiumhydroxid auch wesentlich größer ist als jene von anderen alkalisch wirkenden Lithiumverbindungen, wie Lithiumcyanid. Lithiumcyanid wird beispielhaft als Katalysator auch in der DE-AS 10 85 871 zitiert, jedoch wurde, wie aus dem Vergleichsbeispiel 3 hervorgeht, mit diesem Katalysator nur eine Ausbeute von 51,5 % erzielt.

Die Katalysatormenge beträgt 0,005 bis 5 Mol-%, vorzugsweise 0,1 bis 2 Mol-%, bezogen auf Isophoron. Die Katalysatormenge muß im Falle der Verwendung von mineralsurestabilisiertem flüssigen Cyanwasserstoff mindestens ausreichen, um die Mineralsäure in ein Salz zu überführen und zusätzlich eine katalytisch wirksame Menge LiOH im Reaktionsgemisch aufrechtzuerhalten. Bei Verwendung von mineralsäurefreiem Cyanwasserstoff kann die Katalysatormenge im allgemeinen auf weniger als 1 Mol-% begrenzt werden.

Die mit LiOH katalysierte HCN-Addition an Isophoron wird im allgemeinen bei 100 bis 160 °C durchgeführt, vorzugsweise zwischen 120 und 160 °C. Die Addition verläuft exotherm, so daß die effektive Zugabegeschwindigkeit von Cyanwasserstoff im wesentlichen nur von den apparativen Bedingungen, insbesondere der Kühlerkapazität, abhängt. Eine Nachreaktionszeit ist nicht erforderlich. Das Reaktionsgemisch kann praktisch unmittelbar nach der zügigen Zusammenführung der Reaktionspartner der Aufarbeitung zugeführt werden. Gemäß einer besonders bevorzugten Ausführungsform bringt man das auf etwa 130 °C erwärmte Isophoron-Katalysator-Gemisch unter Aufrechterhaltung einer Temperatur im Bereich zwischen 135 und 150 °C mit Cyanwasserstoff zusammen; das so erhaltene Reaktionsgemisch führt man ohne eine Nachreaktionzeit der Aufarbeitung zu, welche üblicherweise Maßnahmen zur Neutralisation des Katalysators oder zu dessen Abtrennung aus dem Reaktionsgemisch sowie Maßnahmen zur destillativen Trennung des 1,3,3-Trimethyl-5-oxo-carbonitrils von dem Isophoronüberschuß und geringen Mengen Verunreinigungen umfaßt. Das Reaktionsgemisch kann beispielsweise mit wenig Wasser behandelt werden, um die Lithiumverbindungen auszuwaschen; alternativ kann LiOH vor der fraktionierten Destillation mit einer starken Säure, wie beispielsweise Phosphorsäure oder p-Toluolsulfansäure, neutralisiert werden.

Die Erfindung wird anhand der nachstehenden Beispiele und Vergleichsbeispiele verdeutlicht.

### Beispiel 1

In einer Reaktionsapparatur mit Rührer, Kühler, Thermometer und Tropftrichter werden 345 g (367 ml; 2,5 Mol) Isophoron und 0,8 g (0,033 Mol) Lithiumhydroxid vorgelegt. Insgesamt werden 40,5 g (59 ml; 1,5 Mol) flüssiger Cyanwasserstoff zu dem Gemisch bei 135 °C innerhalb von 10 Min. zugegeben. Dabei steigt die Reaktionstemperatur bis ca. 145 °C an. Diese Temperatur wird unter Kühlung gehalten. Anschließend werden 6,8 g (0,036 Mol) p-Toluolsulfonsäure zugegeben. Zur Aufarbeitung wird das überschüssige Isophoron über eine Destillationskolonne im Wasserstrahlvakuum abdestilliert und der Rückstand im Hochvakuum aufgereinigt; es werden erhalten:
- 134,0 g: Isophoron (Sdp₁₅: 89-110 °C)
- 220,5 g: Isophoronnitril (Sdp_{0,1}: 105-110 °C); entsprechend 89,1 % d. Th., bezogen auf eingesetzten Cyanwasserstoff

### Beispiel 2

In einer Reaktionsapparatur - wie in Beispiel 1 beschrieben - werden 345 g (376 ml; 2,5 Mol) Isophoron und 0,8 g (0,033 Mol) Lithiumhydroxid vorgelegt. Von insgesamt 27 g (ca. 39 ml: 1 Mol) fl. Cyanwasserstoff werden ca. 10 ml der Vorlage hinzugefügt und das Gemisch auf 135 °C aufgeheizt. Der restliche Cyanwasserstoff wird über den Tropftrichter innerhalb von 5 Min. zugetropft und die ansteigende Reaktionstemperatur durch Kühlung auf 140 °C gehalten. Danach werden 8 g Toluolsulfonsäure zugegeben und aus dem Reaktionsgemisch über eine Kolonne überschüssiges Isophoron im Vakuum abdestilliert. Der Rückstand wird dann im Hochvakuum aufgereinigt; es werden erhalten:
- 187,1 g: Isophoron (Sdp₁₅: 88-90 °C)
- 155,5 g: Isophoronnitril (Sdp_{0,1:} 105-110 °C), entsprechend 94,2 % d. Th., bezogen auf eingesetzten Cyanwasserstoff

### Beispiel 3

In einer Rührapparatur - wie in Beispiel 1 - werden 276 g (ca. 300 ml; 2 Mol) Isophoron und 0,8 g (0,033 Mol) Lithiumhydroxid vorgelegt und auf 150 °C erhitzt. Innerhalb von 10 Min. werden dann 27 g (39 ml; 1 Mol) fl. Cyanwasserstoff zugetropft; dabei steigt die Reaktionstemperatur auf 168 °C an. Anschließend werden 2,2 ml 85 %ige Phosphorsäure zugegeben und aus dem Reaktionsgemisch das überschüssige Isophoron über eine Kolonne im Vakuum abdestilliert; der Rückstand wird im Hochvakuum aufgereinigt; es werden erhalten:
- 134,6 g: Isophoron
- 155,7 g: Isophoronnitril (Sdp₂: 115-122 °C). entsprechend 94,3 % d. Th. , bezogen auf eingesetzten Cyanwasserstoff

### Beispiel 4

In einer Rührkesselapparatur werden 186 kg (1347,8 Mol) Isophoron und 0,452 kg (18,8 Mol) Lithiumhydroxid vorgelegt und auf 135 °C aufgeheizt. Danach werden unter Kühlung 22,8 kg (844,4 Mol) Cyanwasserstoff so zulaufen lassen, daß die Reaktionstemperatur 145 °C nicht übersteigt. Anschließend werden 3,84 kg p-Toluolsulfonsäure zugegeben und das überschüssige Isophoron im Vakuum über eine Kolonne abdestilliert. Im Hochvakuum wird der Sumpf aufgereinigt. Es werden erhalten:
- 68,5 kg: Isophoron (Sdp₃₀: 118-120 °C)
- 134,0 kg: Isophoronnitril (Sdp₂: 115-120 °C). entsprechend 96,1 % d. Th., bezogen auf eingesetzten Cyanwasserstoff

### Beispiel 5

Beispiel 1 wurde wiederholt, wobei anstelle von 0,8 g (=0,033 Mol) Lithiumhydroxid 0,3 g (=0,012 Mol) Lithiumhydroxid eingesetzt wurden. Die Ausbeute an Isophoronnitril betrug 93,3 %, bezogen auf eingesetzten Cyanwasserstoff. Hierbei wurde nicht mit Säure stabilisierter Cyanwasserstoff eingesetzt.

(In den Beispielen 1 bis 4 und Vergleichsbeispielen 1 bis 3 wurde flüssiger Cyanwasserstoff, der mit ca. 1 Gew.-% Phosphorsäure stabilisiert war, eingesetzt.)

### Vergleichsbeispiel 1

In einer Reaktionsapparatur mit Rührer, Kühler, Thermometer und Tropftrichter werden 345 g (376 ml; 2,5 Mol) Isophoron und 2,2 g (0,033 Mol) Kaliumhydroxid (85 %ig) vorgelegt. Von insgesamt 40,5 g (59 ml; 1,5 Mol) Cyanwasserstoff werden 9 ml der Vorlage hinzugefügt und das Gemisch auf 135 °C erhitzt. Über den Tropftrichter wird der restliche Cyanwasserstoff innerhalb von 3 Stunden zulaufen lassen; dabei fällt die Temperatur auf ca. 110 °C ab und Cyanwasserstoff siedet unter lebhaftem Rückfluß. Nach beendeter Zugabe werden noch 1,5 Stunden nachgerührt, wobei die Temperatur wieder auf ca. 128 °C ansteigt. Anschließend werden 8 g p-Toluolsulfonsäure zugegeben und das überschüssige Isophoron im Wasserstrahlvakuum über eine Kolonne abdestilliert. Der Rückstand wird im Hochvakuum aufgereinigt; es werden erhalten:
- 213 g: Isophoron (Sdp₁₅: 89-95 °C)
- 118 g: Isophoronnitril (Sdp_{0,1}: 100-110 °C), entsprechend 47,6 % d. Th., bezogen auf eingesetzten Cyanwasserstoff

### Vergleichsbeispiel 2

Mengen und Ausführung wie in Vergleichsbeispiel 1, jedoch wird mit 1,3 g (0,033 Mol) Natriumhydroxid katalysiert. Dabei werden erhalten:
- 217,6 g: Isophoron (Sdp₁₅: 89-95 °C)
- 107,3 g: Isophoronnitril (Sdp_{0,1}: 100-107 °C), entsprechend 43,3 % d. Th., bezogen auf eingesetzten Cyanwasserstoff
- 45 g: Rückstand

### Vergleichsbeispiel 3

Gemäß Beispiel 1 wurde die Umsetzung durchgeführt, wobei anstelle LiOH die äquivalente Menge Lithiumcyanid (33 mMol) eingesetzt wurde. Für die Zugabe von Cyanwasserstoff waren 45 Min. erforderlich. Nach einer Nachreaktion von 30 Min. bei 145 °C wurde aufgearbeitet und Isophoronnitril in einer Ausbeute von 51,5 % erhalten.

## Patentansprüche

1. Verfahren zur Herstellung von 1,3,3-Trimethyl-5-oxo-cyclohexan-carbonitril durch Anlagerung von Cyanwasserstoff an Isophoron in Gegenwart einer alkalisch wirkenden Alkaliverbindung als Katalysator bei 100 bis 160 °C,
dadurch gekennzeichnet,
daß man Lithiumhydroxid als Katalysator verwendet und die Umsetzung in Gegenwart von 0,005 bis 5 Mol-% Katalysator, bezogen auf Isophoron, durchführt.

2. Verfahren nach Anspruch 1,
dadurch gekennzeichnet,
daß man Isophoren und Cyanwasserstoff im Molverhältnis 1,1 zu 1 bis 5 zu 1, vorzugsweise 1,5 zu 1 bis 2,5 zu 1, umsetzt und kein weiteres Lösungsmittel verwendet.

3. Verfahren nach Anspruch 1 oder 2,
dadurch gekennzeichnet,
daß man unter Aufrechterhaltung einer Reaktionstemperatur von 135-150 °C flüssigen Cyanwasserstoff mit dem Katalysator enthaltenden Isophoron zusammenbringt und das Reaktionsgemisch ohne Nachreaktionszeit der Aufarbeitung, welche eine Neutralisation oder Abtrennung des Katalysators und Destillation umfaßt, zuführt.

4. Verfahren nach einem der Ansprüche 1 bis 3,
dadurch gekennzeichnet,
daß man die Umsetzung in Gegenwart von 0,1 bis 2 Mol-% Lithiumhydroxid durchführt.

## Claims

1. A process for the preparation of 1,3,3-trimethyl-5-oxo-cyclohexane-carbonitrile by the chemical addition of hydrogen cyanide to isophorone in the presence of an alkali metal compound which has an alkaline action as catalyst at from 100 to 160°C, characterised in that lithium hydroxide is used as catalyst and the reaction is carried out in the presence of from 0.005 to 5 mol-% of catalyst, based on isophorone.

2. A process according to Claim 1, characterised in that isophorone and hydrogen cyanide are reacted in a molar ratio of from 1.1:1 to 5:1, preferably from 1.5:1 to 2.5:1, and no other solvent is used.

3. A process according to Claim 1 or 2, characterised in that liquid hydrogen cyanide is brought together with the catalyst-containing isophorone while the reaction temperature is maintained at 135-150°C and the reaction mixture is then worked up without an after reaction time, this working up comprising a neutralisation or separation of the catalyst and distillation.

4. A process according to one of the Claims 1 to 3, characterised in that the reaction is carried out in the presence of from 0.1 to 2 mol-% of lithium hydroxide.

## Revendications

1. Procédé de préparation de 1,3,3-triméthyl-5-oxo-cyclohexane-carbonitrile par addition d'acide cyanhydrique sur l'isophorone, en présence d'un composé alcalin à effet alcalin comme catalyseur entre 100 et 160°C, caractérisé en ce qu'on utilise l'hydroxyde de lithium comme catalyseur et qu'on réalise la réaction en présence de 0,005 à 5 % molaire de catalyseur, par rapport à l'isophorone.

2. Procédé selon la revendication 1, caractérisé en ce qu'on utilise l'isophorone et l'acide cyanhydrique en proportion molaire de 1,1 à 1 jusqu'à 5 à 1, de préférence de 1,5 à 1 jusqu'à 2,5 à 1 et qu'on n'utilise pas d'autre solvant.

3. Procédé selon la revendication 1 ou 2, caractérisé en ce qu'en maintenant une température de réaction de 135-150°C, on met en contact de l'acide cyanhydrique liquide avec l'isophorone contenant le catalyseur et on traite le mélange réactionnel sans temps de post-réaction, le traitement comprend une neutralisation ou une séparation du catalyseur et la distillation.

4. Procédé selon l'une des revendications 1 à 3, caractérisé en ce qu'on réalise la réaction en présence de 0,1 à 2 % molaire d'hydroxyde de lithium.
